(19) Europäisches Patentamt

European Patent Office

Office européen des brevets

(11) Numéro de publication: **0 230 845 B1**

(12) **FASCICULE DE BREVET EUROPEEN**

(45) Date de publication de fascicule du brevet:
**13.02.91**

(21) Numéro de dépôt: **86430054.6**

(22) Date de dépôt: **24.12.86**

(51) Int. Cl.⁵: **A61H 39/00**, A61N 1/04,
//A61B17/39

(54) **Appareil thérapeutique à décharge d'étincelles.**

(30) Priorité: **31.12.85 FR 8519590**

(43) Date de publication de la demande:
**05.08.87 Bulletin 87/32**

(45) Mention de la délivrance du brevet:
**13.02.91 Bulletin 91/07**

(84) Etats contractants désignés:
**AT BE CH DE ES GB GR IT LI LU NL SE**

(56) Documents cités:
**FR-A- 2 500 745**
**FR-A- 2 563 735**
**US-A- 4 043 342**
**US-A- 4 476 862**

(73) Titulaire: **SOCIETE ANATOMIA - LA BOUTIQUE DU DOS**
**20 rue de Maubeuge**
**F-75009 Paris(FR)**

(72) Inventeur: **DERVIEUX, Dominique**
**170 avenue de Gairaut**
**F-06100 Nice(FR)**

(74) Mandataire: **Madeuf, René Louis et al**
**Cabinet Madeuf Conseils en Propriété Industrielle 3, Avenue Bugeaud**
**F-75116 Paris(FR)**

Rank Xerox (UK) Business Services

## Description

L'invention a pour objet un appareil de soulagement des douleurs et des contractures utilisant des décharges d'étincelles notamment d'origine piézo-électrique dont un des pôles est en contact direct avec la peau.

L'état de la technique peut être défini Par les brevets suivants : FR-A-1 092 420, FR Addition 65.169, DE-A-2 117 917 et les brevets DERVIEUX FR-A-500 745 et FR-A-563 735.

Le brevet DERVIEUX FR-A-500 745 décrit un appareil à électrode unipolaire dont une électrode est branchée à la masse soit par une plaque que tient le patient, soit à une électrode déjà implantée, soit que le patient est en contact avec la poignée qui est conductrice (ce qui présente quelques inconvénients).

Le brevet DERVIEUX FR-A-2 563 735 décrit un appareil selon le préambule de la revendication 1. Cet appareil utilise des électrodes bipolaires de décharge d'étincelles dont leurs propres caractéristiques, la forme, et les caractéristiques de leur support permettent d'être à une certaine distance de la peau par un moyen de séparation isolant.

Cet appareil ne permet pas la décharge d'étincelles sur la peau humide, ni le réglage de l'intensité.

L'appareil selon l'invention tend à améliorer ce dernier appareil qui comprend un générateur piézo-électrique muni d'un n moyen d'actionnement placé dans la poignée de l'appareil et d'au moins une électrode reliée à un pôle dudit générateur, ladite électrode étant une électrode bipolaire qui comprend une ou plusieurs électrodes de décharge.

L'appareil selon la présente invention est caractérisé par le fait que ladite électrode bipolaire comprend une ou plusieurs électrodes métalliques destinées à venir en contact direct avec la peau et que lesdites électrodes de décharge sont reliées à l'autre pôle dudit générateur et qu'elles sont isolées et en retrait par rapport aux électrodes de contact avec la peau permettant la décharge d'étincelles sur la peau par mise à distance automatique de ou des électrodes métalliques de décharge par la ou les électrodes métalliques de contact qui font masse.

L'appareil peut comporter un moyen permettant de faire varier l'intensité des décharges d'étincelles et/ou de faire varier la mise à distance des électrodes de décharge par rapport à la peau :

1° - l'appareil peut comporter un moyen faisant office de butée réglable qui permet de régler la course de la poignée articulée qui actionne le générateur piézo-électrique de type mécanique et permet ainsi une diminution de l'intensité et d'adapter le courant émis à la sensibilité du sujet traité.

2° - l'appareil peut comporter un moyen de mise à distance automatique et réglable de l'électrode de décharge d'étincelles à la peau, ou du plan de contact faisant office d'électrode de contact, soit par un moyen agissant comme une molette ou par un bouton poussoir éventuellement avec levier démultiplicateur ou non, à deux ou multiples positions.

Les dessins ci-joints donnés à titre d'exemples indicatifs et non limitatifs permettront de comprendre aisément l'invention. Ils représentent un mode de réalisation préféré selon l'invention.

La figure 1 est une vue de l'appareil vu de face.

La figure 2 est une vue de l'appareil vu de dos.

La figure 3 est une vue de l'appareil vu de côté.

Les figures 4 à 14 sont des vues des différentes têtes de l'appareil mettant en évidence les caractéristiques notamment de l'électrode de contact.

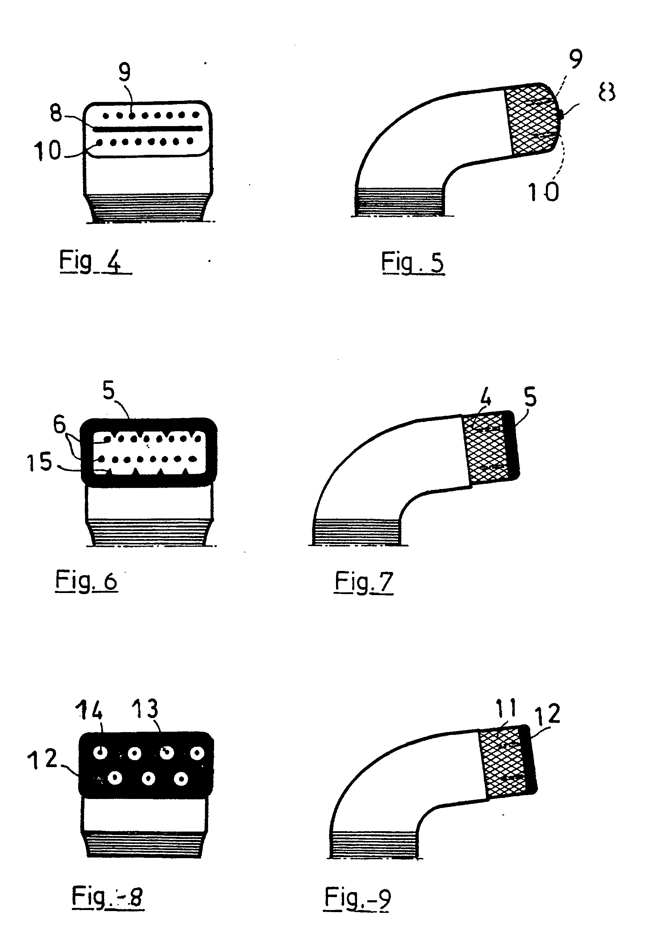

Les figures 4 et 5 sont des vues de face et de côté d'une électrode de contact centrale.

Les figures 6 et 7 sont des vues de face et de côté d'une électrode de contact périphérique.

Les figures 8 et 9 sont des vues de face et de côté d'une électrode de contact à alvéoles.

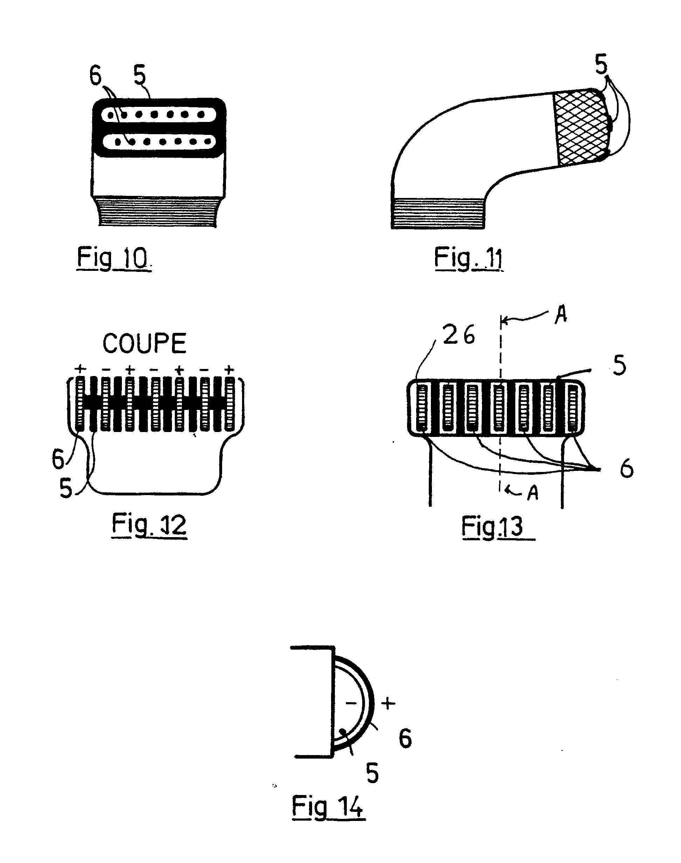

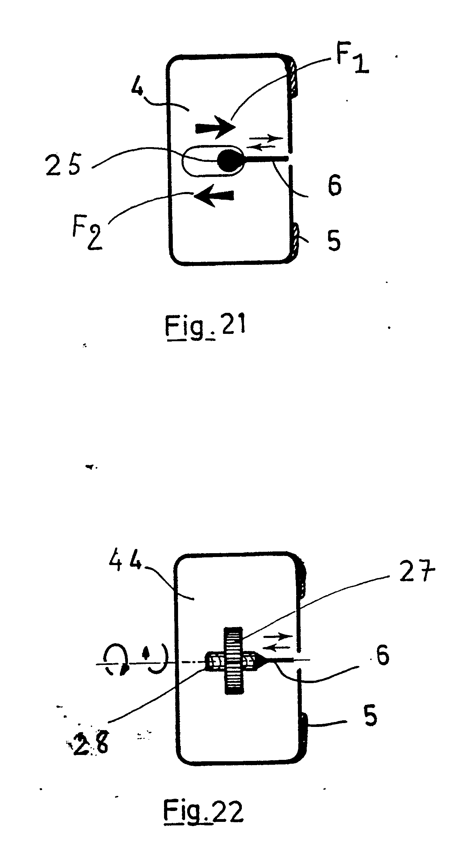

Les figures 10 et 11 représentent un mode de réalisation où l'électrode de contact est une électrode périphérique comme dans la réalisation représentée dans les figures 6 et 7, mais où il y a deux électrodes de décharge d'étincelles 6.

Les figures 12 et 13 représentent un mode de réalisation où les électrodes font office de roulettes dentées de diamétre et de polarité différents (masse en contact de la peau - roulettes à distance).

La figure 14 est une vue de côté selon l'axe AA représenté à la figure 13.

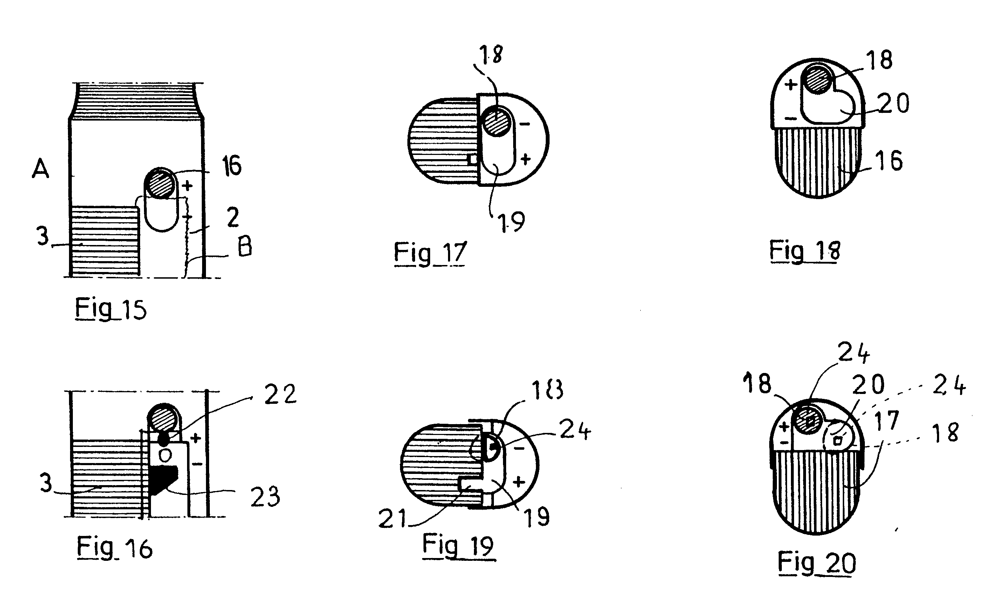

Les figures 15 et 16 représentent un détail de l'appareil au niveau du bouton de réglage de l'intensité.

Les figures 17 et 19 représentent un mode de réalisation où le bouton de réglage se trouve au niveau de l'extrémité du manche (et/ou de la poignée) et où la gorge est longitudinale.

Les figures 18 et 20 représentent un mode de réalisation où le bouton de réglage se trouve au niveau de l'extrémité du manche (et/ou de la poignée) et où la gorge est à baillonette.

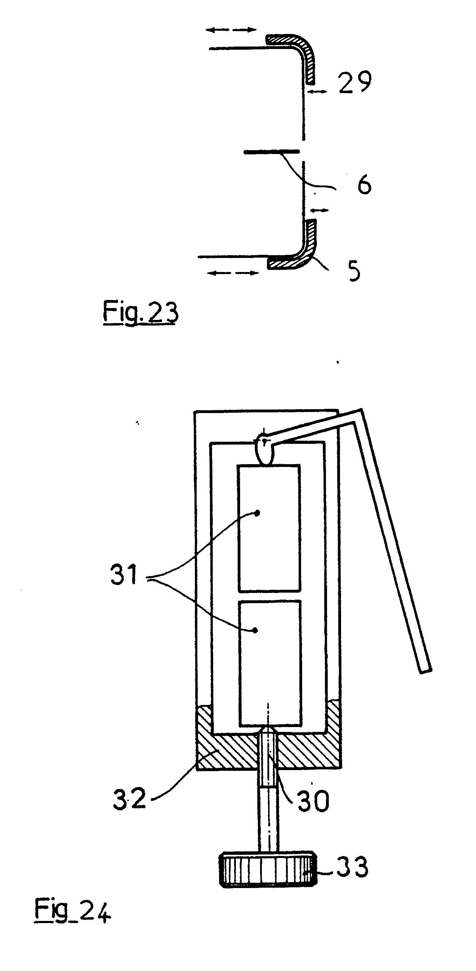

Les figures 21, 22 et 23 sont des vues en coupe de l'appareil mettant en évidence les moyens permettant de régler la distance à la peau de l'électrode de décharge d'étincelle ou du plan faisant office d'électrode de contact. L'un pouvant se déplacer par rapport à l'autre.

L'appareil selon l'invention est composé d'un corps principal 1 qui forme un manche 2 comportant une poignée articulée 3 et une tête, amovible ou pas, 4 qui porte les électrodes bipolaires 5 et 6.

La poignée articulée 3 actionne, de manière mécanique connue, un générateur piézo-électrique.

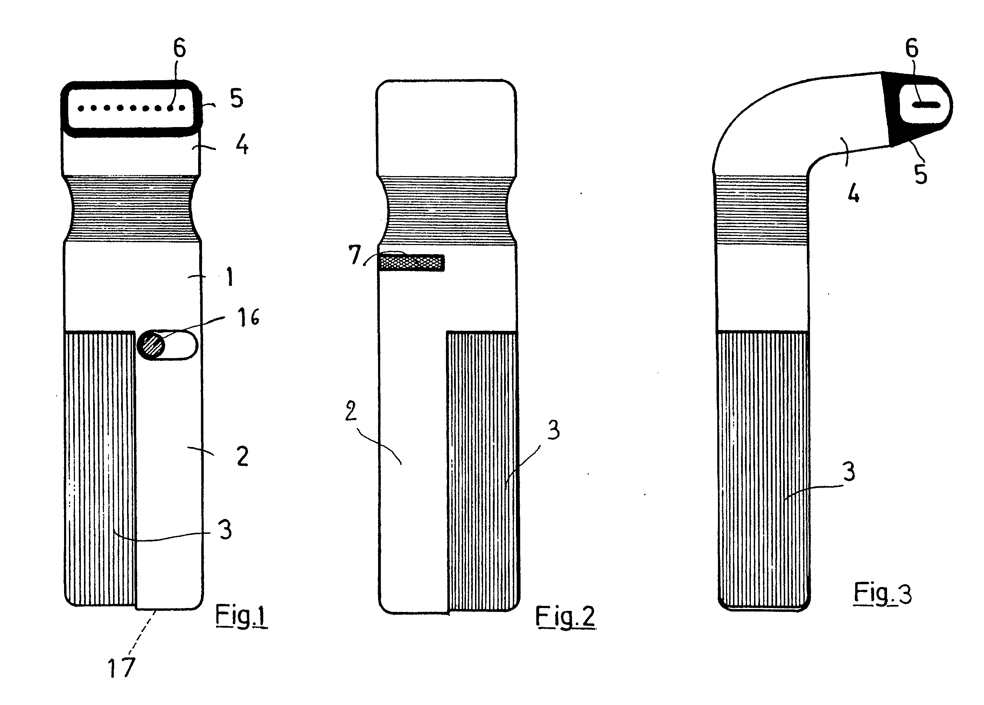

Selon la figure 1, l'appareil comporte une électrode bipolaire qui comprend une électrode métallique 5 de contact direct à la peau. Cette électrode de contact 5 forme dans cet exemple la tête 4 de l'appareil, elle assure un contact direct à la peau, elle est reliée à un pôle.

Une ou plusieurs électrodes de décharges d'étincelles 6 de l'autre pôle sont disposées dans la partie médiane et transversale de la tête 4.

L'électrode de contact 5 direct à la peau maintient automatiquement l'électrode de décharge 6 d'étincelles à distance de la peau (de quelques dixièmes à quelques millimètres de la peau). A cet effet, les pointes de l'électrode de décharge 6 sont plus courtes que la hauteur de la bordure de l'électrode de contact 5 (masse).

Un voyant lumineux 7 permet de s'assurer du bon fonctionnement de l'appareil lorsque l'on appuie et lorsque l'on relache la poignée articulée 3 qui actionne le générateur piézo-électrique.

Dans les figures 4 et 5, une électrode de contact 8 est centrale. Elle forme une barrette de contact 8 qui est disposée dans la partie médiane et transversale de la tête 4 de l'appareil. De part et d'autre de la barrette de contact 8 sont disposées les autres électrodes bipolaires de décharge d'étincelles contre la peau qui forment une rangée 9 et 10 de chaque côté de ladite barrette de contact 8.

Bien entendu, selon un autre mode de réalisation, non représenté sur les figures, il peut y avoir une ou plusieurs électrodes de contact ou de décharge d'étincelles contre la peau.

Les figures 6 et 7 sont des vues de détail des électrodes déjà décrites plus haut (voir les figures 1, 2 et 3) et qui représentent une électrode de contact périphérique 5 et deux électrodes de décharges 6.

Les pointes 15 formées par l'électrode de contact périphérique 5 favorisent les décharges d'étincelles entre une ou plusieurs électrodes de décharges, ici deux rangées 5 et 6 vers lesquelles ces pointes sont dirigées, elles déchargent des étincelles lors du fonctionnement hors contact direct à la peau (à l'air : entre des électrodes ou à travers un tissu sur la peau).

Les figures 8 et 9 sont des vues de détail d'une électrode 11 qui forme une plaque de contact 12 pourvue d'une ou de plusieurs alvéoles 13 dont la partie centrale est occupée par la pointe d'une électrode de décharge 14 d'étincelles.

La pointe de l'électrode 14 est en retrait par rapport au plan de la plaque de contact 12 de l'électrode 11.

Les figures 10 et 11 sont des vues de détail d'une électrode qui forme une plaque de contact 5 pourvue de deux alvéoles dont la partie centrale est occupée par une rangée de pointes de décharges 6 d'étincelles.

L'appareil représenté dans les figures 12, 13 et 14 a une tête composée de roulettes 26, ici dentées et mobiles, de diamètre et de polarité différents :
- masse au contact de la peau par l'électrode de contact à roulette 5 (voir la figure 12);
- électrode de décharge d'étincelles 6 contre la peau composée par des roulettes 26 dentées et mobiles dont le diamètre est légèrement inférieur au diamètre des roulettes de contact 5.

De même, selon un autre mode de réalisation non représenté, nous pourrions n'avoir que des électrodes en arcs de cercle parallèles, fixes ou non, dentées ou non.

Selon les figures 15 et 16 un bouton permet de régler l'intensité de la décharge d'étincelle en faisant varier la course de la poignée articulée 3. Plus la course de la poignée articulée 3 est longue, plus l'intensité est forte. La figure 15 montre un détail de l'appareil au niveau du bouton 16 de réglage de l'intensité. La figure 16 est une vue selon la figure 15 dans laquelle une fenêtre B a été ouverte pour mettre en évidence le mécanisme dudit bouton 16. Le bouton 16 déplace, en fait, une butée 22 qui vient limiter la course de ladite poignée articulée 3 au niveau de sa propre butée 23. La course de ce bouton peut être horizontale.

Selon les figures 17, 18, 19 et 20 le bouton de réglage d'intensité 18 n'est plus au niveau de l'axe d'articulation de la poignée 3, mais à l'extrémité 17 du manche 2 en regard de l'extrémité de la poignée 3. En déplaçant ledit bouton 18, dans une gorge longitudinale 19 (figures 17 et 19) ou à baïonnette 20 (figures 18, 20), on permet un verrouillage de la position choisie, on règle la course de la poignée 3.

Comme dans les figures 15 et 16, le bouton de réglage 18 comporte un ergot 24 qui limite la course de l'extrémité de la poignée 3.

La poignée comporte une fente 21 pour le passage du bouton 18 qui fait office de butée ou d'ergot 24. Lorsque le bouton 18 est déplacé au delà de la fente 21, l'extrémité 16 de la poignée 3 ne peut pas s'enfoncer complétement, la course est donc limitée. Lorsque le bouton 18 est en face de la fente 21, la poignée 3 peut être appuyée à fond, la course est donc maximum.

La figure 21 est une vue en coupe de la tête de l'appareil. Elle met en évidence l'électrode de décharge d'étincelles 6 dont la distance à la peau peut être réglée par un bouton 25 selon les flèches F1, F2. Le bouton 25 peut être solidaire de l'électrode 6 ou être pourvu d'un moyen démultiplicateur permettant un déplacement micrométrique.

De même, on pourrait avoir une électrode de décharge d'étincelle 6 fixe et seul le plan faisant office d'électrode de contact 5 à la peau pourrait se déplacer.

Dans la figure 22, l'électrode de décharge d'étincelle 6 peut se déplacer par rapport à la peau et à l'autre électrode 5 au moyen d'une molette 27 et d'un filetage 28 disposé sur le corps principal de ladite électrode de décharge d'étincelle 6.

Dans la figure 23 l'électrode de contact 5 peut être amovible et selon son épaisseur 29 permettre la mise à distance automatique et variable de l'électrode de décharge d'étincelles 6.

Dans la figure 24, la réglage de l'intensité peut se faire par serrage plus ou moins important des quartz 31, dans l'étrier métallique 32 par la vis de compression 30 ; ce réglage peut être fixe ou effectué à la demande et de façon variable par une molette 33 solidaire ou non de la vis 30, permettant le réglage de l'intensité depuis l'extérieur de l'appareil et le rattrapage de l'usure des quartz. Eventuellement cette disposition permet l'échange standard des quartz.

## Revendications

1. Appareil de soulagement des douleurs et des contractures utilisant des décharges d'étincelles d'origine piézo-électrique en application directe à la peau, comprenant un générateur piézo-électrique muni d'un moyen d'actionnement placé dans la poignée de l'appareil et d'au moins une électrode reliée à un pôle dudit générateur, ladite électrode étant une électrode bipolaire qui comprend une ou plusieurs électrodes de décharge (6, 9, 10, 14), caractérisé par le fait que ladite électrode bipolaire comprend une ou plusieurs électrodes métalliques (5, 8, 11, 12) destinées à venir en contact direct avec la peau et que lesdites électrodes de décharge sont reliées à l'autre pôle dudit générateur et qu'elles sont isolées et en retrait par rapport aux électrodes de contact (5, 8, 11, 12) avec la peau permettant la décharge d'étincelles sur la peau par mise à distance automatique d'une ou des électrodes métalliques de décharge (6, 9, 10, 14) par la ou les électrodes métalliques de contact (5, 8, 11, 12) qui font masse.

2. Appareil selon la revendication 1, caractérisé par le fait qu'il comporte une ou plusieurs électrodes métalliques (5) de contact direct à la peau : cette électrode de contact (5) peut former en fait la tête (4) de l'appareil et mettre automatiquement à distance en retrait par rapport à la peau, une ou plusieurs électrodes de décharges d'étincelles (6) de l'autre pôle, disposées dans la partie médiane et transversale de ladite tête (4).

3. Appareil selon les revendications 1 ou 2, caractérisé par le fait qu'une ou plusieurs électrodes métalliques (5) de contact direct à la peau peuvent comporter des pointes (15) pointées vers les pointes des électrodes de décharge (5) qui favorisent les décharges entre les deux électrodes (5, 6) lors du fonctionnement hors contact direct à la peau.

4. Appareil selon l'une quelconque des revendications 1, 2 ou 3, caractérisé par le fait que la ou les pointes de l'électrode de décharge (6) sont plus courtes que la hauteur de la bordure ou périphérie de tête de l'appareil de l'électrode de contact (5) (masse).

5. Appareil selon l'une quelconque des revendications 1, 2 ou 3, caractérisé par le fait que la ou les électrodes de contact (8) constituant un pôle sont centrales ; elles forment une barrette de contact (8) qui est disposée dans la partie médiane et transversale de la tête (4) de l'appareil ; de part et d'autre de la barrette de contact (8) sont disposées la ou les pointes de décharge d'étincelles de l'autre pôle de l'électrode bipolaire qui forment une ou plusieurs rangées (9 et 10) de chaque côté de ladite barrette de contact (8).

6. Appareil selon l'une quelconque des revendiction ,1, 2 ou 3 caractérisées par le fait que l'électrode de contact (8) constituant un pôle est centrale ; elle forme une ou plusieurs barrettes de contact (8) qui sont disposées dans la partie médiane et transversale de la tête (4) de l'appareil ; d'un côté ou de l'autre de la barrette de contact (8) est disposée l'autre pôle de l'électrode bipolaire de décharge d'étincelles contre la peau qui forme une ou plusieurs rangées de pointes (9 ou 10) parallèle à ladite ou lesdites barrettes de contact (8).

7. Appareil selon la revendication 1 caractérisées par le fait qu'une électrode de contact (11) constituant un des pôles qui forme une ou plusieurs plaques de contact (12), pourvue d'une ou de plusieurs alvéoles (13) dont la partie centrale est occupée par la pointe d'une électrode de décharge (14) d'étincelles ; la pointe de l'électrode de décharge (14) est en retrait par rapport au plan de la plaque de contact (12) de l'électrode de contact (11).

8. Appareil selon la revendication 1 caractérisées par le fait

que les électrodes bipolaires sont formées par des électrodes en arc de cercle parallèles ou sous formes de roulettes (26), dentées ou non, de diamètre et de polarité différents.

9. Appareil selon l'une quelconque des revendications 1 ou 8 caractérisées par le fait

que la tête est composée d'électrodes en arcs de cercle parallèles ou sous forme de roulettes, dentées ou non, de diamètre et de polarité différents, la masse est au contact de la peau par l'électrode de contact (5), l'électrode de décharge d'étincelles (6) contre la peau est en arcs de cercle parallèles ou composée de roulettes dentées dont le diamètre est légèrement inférieur au diamètre des électrodes de contact (5).

10. Appareil selon la revendication 1 caractérisées par le fait

qu'un bouton (16) permet de régler l'intensité de la décharge d'étincelle en faisant varier la course de la poignée articulée (3) ; plus la course de la poignée articulée (3) est longue, plus l'intensité est forte ; le bouton (16) déplace en fait une butée (22 ou 24)qui vient limiter la course de ladite poignée articulée (3) en butant contre sa propre butée (23).

11. Appareil selon l'une quelconque des revendications 1 ou 10 caractérisées par le fait

que le bouton (16) est disposé au niveau de l'axe d'articulation de la poignée (3).

12. Appareil selon l'une quelconque des revendications 1 ou 10 caractérisées par le fait

que le bouton de réglage (18) est situé au niveau de l'extrémité (17) du manche (2) et de la poignée (3).

13. Appareil selon la revendication 1 caractérisées par le fait

qu'elles comportent un moyen de mise à distance automatique et réglable de l'électrode de décharge (6) d'étincelles à la peau, ou du plan de contact (5) faisant office d'électrode de contact soit par un moyen agissant comme une molette (27) ou par un bouton poussoir éventuellement avec levier démultiplicateur ou non, à deux ou multiples positions.

14. Appareil selon l'une quelconque des revendications 1 ou 13 caractérisées par le fait

que l'électrode de décharge d'étincelle (6), dont la distance à la peau peut être réglée par un bouton (25), comporte un bouton (25) qui peut être solidaire de l'électrode (6) ou être pourvu d'un moyen démultiplicateur permettant un déplacement micrométrique.

15. Appareil selon l'une quelconque des revendications 1 ou 13 caractérisées par le fait

que l'électrode de décharge d'étincelle (6) est fixe et seul le plan faisant office d'électrode de contact (5) à la peau peut se déplacer

16. Electrodes selon l'une quelconque des revendications 1 ou 13 caractérisées par le fait

que l'électrode de décharge d'étincelle (6) est mobile et seul le plan faisant office d'électrode de contact (5) à la peau est fixe.

17. Appareil selon la revendication 1 caractérisées par le fait

que l'électrode de contact (5) est amovible et que la mise à distance automatique et variable de l'électrode de décharge d'étincelles (6) se fait au moyen des différentes épaisseurs que peut avoir l'électrode de contact (5).

18. Appareil selon la revendication 1 caractérisées par le fait

que le réglage de l'intensité peut se faire par la variation du serrage des quartz (31) entre-eux au moyen d'un moyen faisant office de presse (32) de serrage commandé de l'extérieur par un moyen de commande (30, 33) (vis, molette).

## Claims

1. Apparatus for relief of pains and contractions using spark discharges of piezo-electric origin in direct application to the skin, comprising a piezo-electric generator provided with an actuating means placed in the handle of the apparatus and at least one electrode connected to one pole of said generator, said electrode being a bipolar electrode which comprises one or several discharge electrodes (6, 9, 10, 14), characterized by the fact that said bipolar electrode comprises one or several metallic electrodes (5, 8, 11, 12) provided to come in direct contact with the skin, and that said discharge electrodes are connected to the other pole of said generator and that they are isolated and recessed relative to the skin-contacting electrodes (5, 8, 11, 12) permitting the discharge of sparks onto the skin by automatically spacing one discharge metallic electrode or electrodes (6, 9. 10, 14) by means of the contact metallic electrode or electrodes (5, 8. 11, 12) that make ground.

2. Apparatus according to claim 1, characterized by the fact that they comprise one or several metallic electrodes (5) for direct contact with the skin ; this contact electrode (5) may form in fact the head (4) of the apparatus and automatically space in recess, relative to the skin, one or several spark-discharging electrodes (6) for the other pole, placed in the median and transverse portion of said head (4).

3. Apparatus according to claims 1 or 2, characterized by the fact that one or several metallic electrodes (5) for direct contact with the skin may comprise points (15) pointed toward the points of the discharge electrodes (5) which favor discharges between the two electrodes (5, 6) at the time of operating outside of direct contact with the skin.

4. Apparatus according to any one of claims 1, 2 or 3, characterized by the fact that the point or points of the discharge electrode (6) are shorter than the height of the border or periphery of head of the apparatus of the contact electrode (5) (ground).

5. Apparatus according to any one of claims 1, 2 or 3. characterized by the fact that the contact electrode or electrodes (8) constituting one pole are centrally disposed ; they form a contact strip (8) which is disposed in the median and transverse portion of the head (4) of the apparatus ; on either side of the contact strip (8) are disposed the spark-discharging point or points of the other pole of the bipolar electrode which form one or several ranges (9 and 10) on each side of said contact strip (8).

6. Apparatus according to any one of claims 1, 2 or 3, characterized by the fact that the contact electrode (8) constituting one pole is centrally disposed ; it forms one or several contact strips (8) which are disposed in the median and transverse portion of the head (4) of the apparatus ; on either side of the contact strip (8) is disposed the other pole of the bipolar electrode for discharging sparks against the skin which forms one or several ranges of points (9 or 10) parallel to said contact strip or strips (8).

7. Apparatus according to claim 1, characterized by the fact that a contact electrode (11) constituting one of the poles which forms one or several contact plates (12), provided with one or several small cavities (13) the central portion of which is occupied by the point of a spark-discharging electrode (14) ; the point of the discharge electrode (14) is recessed relative to the plane of the contact plate (12) of the contact electrode (11).

8. Apparatus according to claim 1, characterized by the fact that the bipolar electrodes are formed by parallel, circular arc-shaped electrodes or in the form of rollers (26), toothed or not, of different diameter and polarity.

9. Apparatus according to any one of claims 1 or 8, characterized by the fact that the head is composed of parallel, circular arc-shaped electrodes or electrodes in the form of rollers, toothed or not, having different diameter and polarity, electrical ground is in contact with the skin via the contact electrode (5) , the electrode (6) for discharging sparks against the skin is in the form of parallel circular arcs or composed of toothed rollers, the diameter of which is slightly less than the diameter of the contact electrodes (5).

10. Apparatus according to claim 1, characterized by the fact that a button (16) permits adjusting the intensity of the discharge of sparks by causing the path of the articulated handle (3) to be varied ; the longer is the path of the articulated handle (3), the greater is the intensity ; the button (16) displaces in fact an abutment (22 or 24) which limits the path of said articulated handle (3) by abutting against its own abutment (23).

11. Apparatus according to any one of claims 1 or 10, characterized by the fact that the button (16) is disposed at the level of the articulation axis of the handle (3).

12. Apparatus according to any one of claims 1 or 10, characterized by the fact that the regulating button (18) is disposed at the end (17) of the sleeve (2) and of the handle (3).

13. Apparatus according to claim 1, characterized by the fact that it comprises a means for automatically and adjustably spacing the spark-discharging electrode (6) from the skin, or from the plane of contact (5) serving as the contact electrode either by means acting as a threaded wheel (27) or by a push button possibly with lever having a gear reduction or not with two or more positions.

14. Apparatus according to any one of claims 1 or 13, characterized by the fact that the spark-discharging electrode (6), the distance of which from the skin may be adjusted by a button (25), comprises a button (25) which may be rigidly connected to the electrode (6) or may be provided with a gear reduction means permitting micrometric displacement.

15. Apparatus according to any one of claims 1 or 13, characterized by the fact that the spark-discharging electrode (6) is fixed, and only the plane serving as the electrode (5) for contacting the skin may be displaced.

16. Apparatus according to any one of claims 1 or 13, characterized by the fact that the spark-discharging electrode (6) is movable and only the plane serving as the electrode (5) for contacting the skin is fixed.

17. Apparatus according to claim 1, characterized by the fact that the contact electrode (5) is removable and that the automatic and variable spacing of the spark-discharging electrode (6) is effected by means of different thicknesses which the contact electrode (5) may have.

18. Apparatus according to claim 1, characterized by the fact that the adjustment of the intensity may be effected by varying the clamping force of the crystals (31) therebetween by means of a means serving as a clamping press (32) exteriorly controlled by a control means (30, 33) (screw, threaded wheel).

**Ansprüche**

1. Apparat zur Linderung Von Schmerzen und Verspannungen unter Ausnutzung von direkt auf der Haut angewendeten Funkenentladungen piezo-elektrischen Ursprunges, umfassend einen piezo-elektrischen Generator, welcher mit einer Betätigungseinrichtung versehen und im Handgriff des Apparates angeordnet ist, sowie zumindest eine, mit einem Pol des besagten Generators verbundenen Elektrode, wobei diese Elektrode eine bipolare Elektrode ist, und eine oder mehrere Entladungselektroden (6, 9, 10, 14) umfaßt, dadurch gekennzeichnet, daß die besagte bipolare Elektrode eine oder mehrere metallische Elektroden (5, 8, 11, 12) umfaßt, welche zum direkten Kontakt mit der Haut bestimmt sind, und daß die besagten Entladungselektroden mit dem anderen Pol des besagten Generators verbunden sind, sowie daß sie gegenüber den Elektroden (5, 8, 11, 12) zum Kontakt mit der Haut isoliert und zurückversetzt sind, und daß durch die automatische Einstellung des Abstandes der metallischen Entladungselektrode(n) (6, 9, 10, 14) gegenüber der oder den die Masse darstellenden metallischen Kontakt-Elektrode(n) (5, 8, 11, 12) die Funkenentladung auf der Haut erfolgt.

2. Apparat gemäß Anspruch 1, dadurch gekennzeichnet, daß er eine oder mehrere metallische Elektroden (5) für den direkten Kontakt mit der Haut umfaßt, wobei diese Kontaktelektrode (5) den Kopf (4) des Apparates bilden kann und durch Zurückweichen in Bezug auf die Haut automatisch die Entfernung zu einer oder mehreren im mittleren Bereich und quer im besagten Kopf (4) angeordneten Funkenentladungs-Elektroden (6) einstellen kann.

3. Apparat gemäß Anspruch 1 oder 2, dadurch gekennzeichnet, daß eine oder mehrere metallische Elektroden (5) für den direkten Kontakt mit der Haut Spitzen (15) aufweisen, welche auf die Spitzen der Entladungselektroden (5) hin zeigen und die Entladung zwischen den beiden Elektroden (5,6) während des Betriebs erleichtern, wenn kein direkter Hautkontakt gegeben ist.

4. Apparat gemäß einem der Ansprüche 1 bis 3, dadurch gekennzeichnet, daß die Spitze(n) der Entladungselektrode (6) kürzer ist (sind) als die Höhe der Umfassung oder des äußeren Teiles des Kopfes des Apparates der Kontaktelektrode (Masse)(5).

5. Apparat gemäß einem der Ansprüche 1 bis 3, dadurch gekennzeichnet, daß die Kontaktelektrode(n) (8), welche einen Pol darstellen, zentral angeordnet sind und einen Kontaktstab (8) bilden, welcher im mittleren Bereich und quer im Kopf (4) des Apparates verlaufend angeordnet ist, und wobei zu beiden Seiten des Kontaktstabes (8) die Spitze(n) der Funkenentladung des anderen Poles der bipolaren Elektrode angeordnet sind, und somit eine oder mehrere Reihen (9 und 10) auf beiden Seiten des besagten Kontaktstabes (8) bilden.

6. Apparat gemäß einem der Ansprüche 1 bis 3, dadurch gekennzeichnet, daß die einen Pol darstellende Kontaktelektrode (8) zentral angeordnet ist, wobei sie einen oder mehrere Kontaktstäbe (8) bildet, welche im mittleren Teil und quer im Kopf (4) des Apparates verlaufend, angeordnet sind, wobei zu beiden Seiten des Kontaktstabes (8) der andere Pol der bipolaren Funkenentladungselektrode zur Haut angeordnet ist und eine oder mehrere parallel zu dem oder den besagten Kontaktstäben (8) verlaufende Reihen von Spitzen (9 oder 10) bildet.

7. Apparat gemäß Anspruch 1, dadurch gekennzeichnet, daß eine einen der Pole darstellende Kontaktelektrode (11) einen oder mehrere Kontaktplatten (12) bildet, welche mit einer oder mehreren Zellen (13) versehen sind, in deren zentralen Bereich die Spitze einer Funkenentladungselektrode (14) angeordnet ist, wobei die Spitze der Entladungselektrode (14) in Bezug auf die Ebene der Kontaktplatte (12) der Kontaktelektrode (11) zurückversetzt ist.

8. Apparat gemäß Anspruch 1, dadurch gekennzeichnet, daß die bipolaren Elektroden durch parallele und kreisbogenförmige oder rollenförmige Elektroden (26), gezähnt oder ungezähnt, mit unterschiedlichem Durchmesser und unterschiedlicher Polarität gebildet sind.

9. Apparat gemäß einem der Ansprüche 1 oder 8, dadurch gekennzeichnet, daß der Kopf aus parallelen kreisbogenförmigen oder rollenförmigen Elektroden, gezähnt oder ungezähnt, und mit unterschiedlichem Durchmesser und unterschiedlicher Polarität, zusammengesetzt ist, wobei die Masse mit der Haut über die Kontaktelektrode (5) in Kontakt ist und die Funkenentladungselektrode (6) aus parallelen Kreisbögen oder gezähnten Rollen zusammengesetzt ist, deren Durchmesser geringfügig kleiner ist als der Durchmesser der Kontaktelektroden (5).

10. Apparat gemäß Anspruch 1, dadurch gekennzeichnet, daß ein Knopf (16) eine Einstellung der Intensität der Funkenentladung durch Veränderung des Weges des beweglichen Handgriffes (3) erlaubt, wobei die Intensität umso stärker wird, je größer der Weg des beweglichen Handgriffes (3) ist, und wobei der Knopf (16) einen Anschlag (22 oder 24) verschiebt, welcher den Weg des besagten, beweglichen Handgriffes (3) durch Abstützen gegen dessen eigenen Anschlag (23) beschränkt.

11. Apparat gemäß einem der Ansprüche 1 oder 10, dadurch gekennzeichnet, daß der Knopf (16) in Höhe der Drehachse des Handgriffes (3) angeordnet ist.

12. Apparat gemäß einem der Ansprüche 1 oder 10, dadurch gekennzeichnet, daß der Einstellknopf (18) in Höhe des Endstückes (17) des Stieles (2) und des Handgriffes (3) angeordnet ist.

13. Apparat gemäß Anspruch 1, dadurch gekennzeichnet, daß er eine regelbare und automatische Einrichtung zur Einstellung des Abstandes der Funkenentladungselektrode (6) zur Haut oder zu der als Kontaktelektrode dienenden Kontaktebene (5) umfaßt, wobei diese Einrichtung als Einstellrädchen (27) oder als Druckknopf, allenfalls mit einem Untersetzungshebel, mit zwei oder mehreren Stellungen, ausgebildet sein kann.

14. Apparat gemäß einem der Ansprüche 1 oder I3, dadurch gekennzeichnet, daß die Funkenentladungselektrode (6) einen Knopf (25) umfaßt, welcher mit der Elektrode (6) verbunden sein kann oder mit einer eine Feinstverstellung ermöglichenden Untersetzungseinrichtung versehen sein kann, wobei der Abstand der Elektroden (6) zur Haut durch den Knopf (25) einstellbar ist.

15. Apparat gemäß einem der Ansprüche 1 oder 13, dadurch gekennzeichnet, daß die Funkenentladungselektrode (6) fix ist und lediglich die als Kontaktelektrode (5) zur Haut dienende Ebene verschiebbar ist.

16. Apparat gemäß einem der Ansprüche 1 oder 13, dadurch gekennzeichnet, daß die Funkenentladungselektrode (6) beweglich ist, und lediglich die als Kontaktelektrode (5) zur Haut dienende Ebene fix ist.

17. Apparat gemäß Anspruch 1, dadurch gekennzeichnet, daß die Kontaktelektrode (5) abnehmbar ist, und die automatische und veränderliche Einstellung des Abstandes der Funkenentladungselektrode (6) durch unterschiedliche Dicke der Kontaktelektrode (5) erzielbar ist.

18. Apparat gemäß Anspruch 1, dadurch gekennzeichnet, daß die Einstellung der Intensität durch eine Veränderung der Klemmung der Quarze (31) zwischeneinander mit Hilfe einer als Klemmpresse (32) wirkenden Einrichtung durchführbar ist, welche von außen durch eine Einstelleinrichtung. (Schraube, Rädchen) (30, 33) einstellbar ist.

Fig.1

Fig.2

Fig.3

Fig. 4

Fig. 5

Fig. 6

Fig. 7

Fig.-8

Fig.-9

Fig 10.

Fig. 11

COUPE

Fig. 12

Fig.13

Fig 14

Fig. 21

Fig. 22

EP 0 230 845 B1

Fig. 15

Fig. 16

Fig. 17

Fig. 18

Fig. 19

Fig. 20

13

Fig. 23

Fig. 24